# EUROPÄISCHE PATENTSCHRIFT

(11) **EP 0 455 785 B1**
(45) Veröffentlichungstag und Bekanntmachung des Hinweises auf die Patenterteilung: **31.01.1996**
(21) Anmeldenummer: 90917566.3
(22) Anmeldetag: 23.11.1990
(51) Int. Cl.: C07K 14/81, A61K 38/55

(54) **LEUKOZYTENELASTASE UND KATHEPSIN G BLOCKIERENDES PEPTID**
LEUCOCYTE ELASTASIS AND CATHEPSIN G-BLOCKING PEPTIDE
PEPTIDE A EFFET DE BLOCAGE DE LA LEUCOZYTENELASTASE ET DE LA CATHEPSINE G

(30) Priorität: 24.11.1989 DE 3938971
(43) Veröffentlichungstag der Anmeldung: 13.11.1991
(73) Patentinhaber: Gesellschaft für Biotechnologische Forschung mbH (GBF), D-38124 Braunschweig (DE)
(72) Erfinder: COLLINS, John, Prof.Dr., D-3300 Braunschweig (DE); TAUBE, Wilfried, D-3300 Braunschweig (DE); FINK, Edwin, Prof.Dr., D-8000 München 2 (DE); FRITZ, Hans, Prof.Dr., D-8000 München 2 (DE)
(74) Vertreter: Boeters, Hans Dietrich, Dr.
(86) Internationale Anmeldenummer: EP9002004
(87) Internationale Veröffentlichungsnummer: WO9108228

(56) Entgegenhaltungen:
- EP-A- 0 252 057
- EP-A- 0 346 500
- FEBS Letters, Band 270, Nr. 1,2, September 1990, Elsevier Science Publishers B. V. (Biomedical Division), Federation of European Biochemical Societies, E. Fink et al.: "Almino acid sequence elucidation of human acrosin- trypsin inhibitor (HUSI.II) reveals that Kazal-type proteinase inhibitors are structurally related to beta-subunits of glycoprotein hormones", Seiten 222-224
- Methods Enzymology, Band 45, 1976, H. Schiessler et al.: "Acid-stable proteinase inhibitors from human seminal plasma", Seiten 847-859

## Beschreibung

### Leukozytenelastase und Kathepsin G blockierendes Peptid, DNA, Vektor, Wirtsorganismus und Verfahren zu seiner Gewinnung sowie pharmazeutisches Präparat

Lysosomale Proteasen, die in den Extrazellulärraum gelangen, werden normalerweise von endogenen Proteinase-Inhibitoren rasch inaktiviert, beispielsweise von α₁-Proteinase-Inhibitor; vgl. Ann. Rev. Biochem., 52 (1983), 655. In Situationen, die mit massiver Freisetzung lysosomaler Proteasen verbunden sind, beispielsweise bei akuten und chronischen Entzündungen, Polytrauma oder Schock, kann eine lokale oder allgemeine Überlastung dieses Schutzmechanismus die Folge sein. Die Konsequenz kann darin bestehen, daß Bindegewebe und Plasmaproteine einschließlich Gerinnungs-, Fibrinolyse- und Komplementfaktoren in starkem Maße durch Elastase und andere lysosomale Proteasen abgebaut werden, was zu schweren klinischen Symptomen führen kann, beispielsweise zu Emphysemen, Schocklunge, ARDS, Gerinnungsstörungen, Nieren- und Leberversagen. Als möglicher therapeutischer Ansatz kommt die Blockierung der überschießenden proteolytischen Aktivität durch solche Inhibitoren in Frage, die spezifisch gegen die lysosomalen Proteasen Leukozytenelastase (neutrophile Elastase) und Kathepsin G gerichtet sind.

In experimentellen Modellen wurde bereits der therapeutische Nutzen von synthetischen und tierischen Elastase-Inhibitoren bei Sepsis und Emphysem nachgewiesen; vgl. beispielsweise Am. Rev. Respir. Dis., 127 (1983) 554-558 und Eur. J. Respir. Dis., 66 Suppl. 139 (1985) 66-70). Für den Einsatz in der Humanmedizin sind jedoch Protease-Inhibitoren menschlichen Ursprungs vorzuziehen, um toxische und allergene Nebenwirkungen zu vermeiden. Dies gilt insbesondere für Langzeittherapien wie die Behandlung eines α₁-Proteinase-Inhibitor-Mangels.

α₁-Proteinase-Inhibitor selbst, der natürliche Gegenspieler der neutrophilen Elastase, ein Glykoprotein mit einer Molekülmasse von 53000 Dalton, ist für eine therapeutische Anwendung wenig geeignet. Wegen der hohen Molekülmasse wäre der Einsatz großer Gewichtsmengen des Inhibitors erforderlich. Zwar wären die erforderlichen Mengen des Proteins auf gentechnischem Wege zugänglich, jedoch nicht in der korrekt glykosylierten Form. Da die biologische Halbwertszeit nicht korrekt modifizierter rekombinanter Proteine in der Zirkulation drastisch vermindert ist (vgl. beispielsweise J. Biol. Chem., 251 (1986) 10404), wäre es erforderlich, noch größere Inhibitormengen einzusetzen. Zudem bestünde die Gefahr allergischer Reaktionen.

Der Mucus-Proteinase-Inhibitor (MPI) ist in einer Reihe muköser Sekrete des menschlichen Organismus enthalten; vgl. beispielsweise Schießler et al. in: Havemann & Janoff, Neutral Proteases of Human Polymorphonuclear Leukocytes: 197-207. Urban & Schwarzenberg, Baltimore, 1978; FEBS Lett., 199 (1986) 43-48; Biol. Chem. Hoppe-Seyler, 369 Suppl. (1988) 79-82). MPI hat eine Molekülmasse von 14000 Dalton und besteht aus zwei aktiven Domänen, wobei die eine Domäne gegen Elastase und die andere Domäne gegen Trypsin und trypsinähnliche Enzyme gerichtet ist. Dieser Inhibitor ist daher nicht spezifisch für Elastase.

Da spezifische Proteinase-Inhibitoren mit deutlich kleinerer Molekülmasse als der des α₁-Proteinase-Inhibitors und mit erwiesener in-vivo-Schutzfunktion gegen Leukozytenelastase und Kathepsin G beim Menschen bisher nicht bekannt sind, müssen solche Inhibitoren durch Modifikation bereits bekannter menschlicher Inhibitoren maßgeschneidert werden, also durch gezielten Austausch einzelner Aminosäuren durch andere, vorzugsweise natürliche Aminosäurereste, spezifische Hemmstoffe für Leukozytenelastase und Kathepsin G hergestellt werden. Um bei klinischer Anwendung derartiger modifizierter Inhibitoren antigene Reaktionen zu vermeiden, muß andererseits die Anzahl der ausgetauschten Aminosäuren so niedrig wie möglich gehalten werden.

Die vorliegende Erfindung geht nun vom Protease-Inhibitor HUSI-II aus, der im menschlichen Seminalplasma enthalten ist, eine niedrige Molekülmasse von 6900 Dalton besitzt und spezifisch Akrosin und Trypsin hemmt.

Gemäß einer Ausführungsform der Erfindung wird
(a) ein Peptid der folgenden Aminosäuresequenz (I) oder
(b) ein Peptid umfassend die folgende Aminosäuresequenz (I) oder
(c) ein Peptid der folgenden Aminosäuresequenz (I) oder umfassend die folgende Aminosäuresequenz (I), bei der eine oder mehrere Aminosäuren weggelassen sind, oder
(d) ein Peptid der folgenden Aminosäuresequenz (I) oder umfassen die folgende Aminosäuresequenz (I) vorgesehen, bei der eine oder mehrere Aminosäuren jeweils gegen eine andere Aminosäure ausgetauscht sind: 
10
Met Ala Leu Ser Val Leu Arg Leu Ala Leu Leu Leu Leu Ala Val

20 30
Thr Phe Ala Ala Ser Leu Ile Pro Gln Phe Gly Leu Phe Ser Lys

40
Tyr Arg Thr Pro Asn Cys Ser Gln Tyr Arg Leu Pro Gly Cys Pro

50 60
Arg His Phe Asn Pro Val Cys Gly Ser Asp Met Ser Thr Tyr Ala

70
Asn Glu Cys Thr Leu Cys Met Lys Ile Arg Glu Gly Gly His Asn

80
Ile Lys Ile Ile Arg Asn Gly Pro Cys (I)
wobei das Peptid Leukozytenelastase und Kathepsin G blockiert.

Das Peptid (a) kann nach dem im folgenden näher beschriebenen erfindungsgemäßen Verfahren in - gegenüber dem Stand der Technik - besonders reiner Weise gewonnen werden.

Bei dem Peptid (b), (c) oder (d) kann der Aminosäuresequenz (I) ein Leader-Peptid verangehen, beispielsweise eine Signalsequenz oder eine Signalsequenz mit Linkersequenz.

Bei dem Peptid gemäß (c) und (d) kann die Modifikation etwa bis zu 43 Aminosäuren, vorzugsweise bis zu 38 Aminosäuren betreffen.

So kann bei dem Peptid gemäß (c) die Aminosäure der Position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 und/oder 32 der angeführten Aminosäuresequenz (I) weggelassen sein. Bei dem Peptid gemäß (d) kann so die Aminosäure der Position 45, 46, 47, 48, 49, 56, 57, 60, 64 und/oder 67 durch eine andere Aminosäure, vorzugsweise eine andere in der Natur vorkommende Aminosäure ersetzt sein.

Gemäß einer weiteren Ausführungsform betrifft die Erfindung eine DNA, die
(a') ein Peptid gemäß (a) kodiert und die folgenden Basensequenz (II) oder eine funktionell äquivalente Basensequenz aufweist oder
(b') ein Peptid gemäß (b), (c) oder (d) kodiert und die folgende Basensequenz (II) oder eine funktionell äquivalente Basensequenz umfaßt oder
(c') ein Peptid gemäß (c) kodiert und die folgende Basensequenz (II), bei der ein oder mehrere Kodons weggelassen sind, oder eine funktionell äquivalente Basensequenz aufweist oder umfaßt, oder
(d') ein Peptid gemäß (d) kodiert und die folgende Basensequenz (II), bei der ein oder mehrere Kodons jeweils gegen ein anderes Kodon ausgetauscht sind, oder eine funktionell äquivalente Basensequenz aufweist oder umfaßt:

70 80 90 100 110 120
ATGGCGCTGTCGGTGCTGCGCTTGGCGCTGCTGCTCCTGGCAGTCACCTTCG
M A L S V L R L A L L L L A V T F

130 140 150 160 170 180
CAGCCTCTCTGATCCCTCAATTTGGTCTGTTTTCAAAATATAGAACGCCAAACTGCTCTC
A A S L I P Q F G L F S K Y R T P N C S
190 200 210 220 230 240
AGTATAGATTACCAGGATGTCCCAGACACTTTAACCCTGTGTGTGGCAGTGACATGTCCA
Q Y R L P G C P R H F N P V C G S D M S

250 260 270 280 290 300
CTTATGCCAATGAATGTACTCTGTGCATGAAAATCAGGGAAGGTGGTCATAATATTAAAA
T Y A N E C T L C M K I R E G G H N I K

310 320
TCATTCGAAATGGACCCTGC (II)
I I R N G P C

Unter funktionell äquivalenten Sequenzen werden solche DNA-Sequenzen verstanden, die trotz unterschiedlicher Sequenz dasselbe Protein kodieren, da bekanntermaßen in einigen Kodons ein, zwei oder drei Basen ausgetauscht werden können, ohne daß sich in dem kodierten Protein die betreffende Aminosäure ändert.

Die Basensequenz (II) oder die dazu funktionell äquivalente Basensequenz kann am 5'-Terminus um Kodons für ein Leader-Peptid, beispielsweise für eine Signalsequenz oder eine Signalsequenz mit Linkersequenz, verlängert sein.

Bei der Basensequenz (II) oder der dazu funktionell äquivalenten Basensequenz kann das Kodon für die Aminosäure der Position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 und/oder 32 der Sequenz (I) weggelassen sein.

Bei der Basensequenz (II) oder der dazu funktionell äquivalenten Basensequenz kann das Kodon für die Aminosäure der Position 45, 46, 47, 48, 49, 56, 57, 60, 64 und/oder 67 durch ein Kodon für eine andere Aminosäure, vorzugsweise eine andere in der Natur vorkommende Aminosäure, ersetzt sein.

Gemäß einer weiteren Ausführungsform betrifft die vorliegende Erfindung auch einen Vektor zur Expression eines der vorstehend angeführten Peptide, wobei der Expressionsvektor eine der vorstehend angeführten DNA-Sequenzen umfaßt. Ein derartiger Vektor kann mit Hilfe von pMAMPF2 bzw. pMAMPF3 vorgesehen werden.

Gemäß einer weiteren Ausführungsform betrifft die Erfindung einen Wirtsorganismus, beispielsweise Escherichia coli, der mit einem erfindungsgemäßen Expressionsvektor transformiert ist.

Gemäß einer weiteren Ausführungsform betrifft die Erfindung ein Verfahren zur Gewinnung eines erfindungsgemäßen Peptids, bei dem man einen erfindungsgemäßen Wirtsorganismus verwendet und das Peptid in an sich bekannter Weise gewinnt.

Der Vorteil der erfindungsgemäßen Peptide besteht darin, daß sie spezifisch Leukozytenelastase hemmen und daß die Komplexe, die diese Peptide mit Leukozytenelastase bilden, aufgrund ihrer relativ niedrigen Molekülmasse die Niere passieren können, so daß eine effektive Eliminierung der freigesetzten Elastase gewährleistet ist. Da sich die erfindungsgemäßen Peptide an HUSI-II menschlichen Ursprungs orientieren und eine niedrige Molekülmasse besitzen, darf man davon ausgehen, daß man bei klinischer Anwendung nicht mit Komplikationen infolge Abwehrreaktionen des Immunsystems zu rechnen hat.

So werden gemäß einer Ausführungsform der Erfindung schließlich pharmazeutische Präparate vorgesehen, die aus einem erfindungsgemäßen Peptid bestehen oder ein derartiges Peptid ggf. neben einem oder mehreren anderen Wirkstoffen und/oder einem üblichen Träger und/oder Verdünnungsmittel umfassen.

Nachstehend wird die Erfindung durch ein Beispiel näher erläutert.

### Beispiel 1

Die DNA-Sequenz der Formel (II)wurde aus dem Plasmid pTZ19R-HUSI II in einen zugänglichen Expressionsvektor, beispielsweise pMAMPF3 (M. Szardenings, Thesis TU Braunschweig 1989 und Szardenings & Collins in Gene, 94 (1990) 1 bis 7) überführt, mit dem ein käuflicher E.-coli-Stamm, beispielsweise K12 JM103 (Pharmacia LKB No. 27-1511-o1; Messing et al., Nucleic Acids Research, 9 (1981) 309), transformiert wurde. Das Plasmid pTZ19R-HUSI II ist bei der Deutschen Sammlung von Mikroorganismen als DSM 5627 hinterlegt. Durch Kultivierung des Wirtsorganismus ließ sich das Peptid der Aminosäuresequenz (I) gewinnen.

## Patentansprüche

1. (a) Peptid der folgenden Aminosäuresequenz (I) oder
(b) Peptid umfassend die folgende Aminosäuresequenz (I) oder
(c) Peptid der folgenden Aminosäuresequenz (I) oder umfassend die folgende Aminosäuresequenz (I), bei der eine oder mehrere Aminosäuren weggelassen sind oder
(d) Peptid der folgenden Aminosäuresequenz (I) oder umfassend die folgende Aminosäuresequenz (I), bei der eine oder mehrere Aminosäuren jeweils gegen eine andere Aminosäure ausgetauscht sind: 
10
Met Ala Leu Ser Val Leu Arg Leu Ala Leu Leu Leu Leu Ala Val
20 30
Thr Phe Ala Ala Ser Leu Ile Pro Gln Phe Gly Leu Phe Ser Lys
40
Tyr Arg Thr Pro Asn Cys Ser Gln Tyr Arg Leu Pro Gly Cys Pro
50 60
Arg His Phe Asn Pro Val Cys Gly Ser Asp Met Ser Thr Tyr Ala
70
Asn Glu Cys Thr Leu Cys Met Lys Ile Arg Glu Gly Gly His Asn
80
Ile Lys Ile Ile Arg Asn Gly Pro Cys (I)
wobei das Peptid Leukozytenelastase und Kathepsin G blockiert.

2. Peptid nach Anspruch 1 (b), (c) oder (d), dadurch gekennzeichnet, daß der Aminosäuresequenz (I) ein Leader-Peptid vorangeht, beispielsweise eine Signalsequenz oder eine Signalsequenz mit Linkersequenz.

3. Peptid nach Anspruch 1 (c), dadurch gekennzeichnet, daß die Aminosäure der Position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 und/oder 32 der angeführten Aminosäuresequenz (I) weggelassen ist.

4. Peptid nach Anspruch 1 (d), dadurch gekennzeichnet, daß die Aminosäure der Position 45, 46, 47, 48, 49, 56, 57, 60, 64 und/oder 67 durch eine andere Aminosäure, vorzugsweise eine andere in der Natur vorkommende Aminosäure, ersetzt ist.

5. DNA, dadurch *gekennzeichnet,* daß sie
(a') ein Peptid gemäß Anspruch 1 (a) kodiert und die folgende Basensequenz (II) oder eine funktionell äquivalente Basensequenz aufweist oder
(b') ein Peptid gemäß Anspruch 1 (b), (c) oder (d) kodiert und die folgende Basensequenz (II) oder eine funktionell äquivalente Basensequenz umfaßt oder
(c') ein Peptid gemäß Anspruch 1 (c) kodiert und die folgende Basensequenz (II), bei der ein oder mehrere Kodons weggelassen sind, oder eine funktionell äquivalente Basensequenz aufweist oder umfaßt oder
(d') ein Peptid gemäß Anspruch 1 (d) kodiert und die folgende Basensequenz (II), bei der ein oder mehrere Kodons jeweils gegen ein anderes Kodon ausgetauscht sind, oder eine funktionell äquivalente Basensequenz aufweist oder umfaßt:

70 80 90 100 110 120
ATGGCGCTGTCGGTGCTGCGCTTGGCGCTGCTGCTCCTGGCAGTCACCTTCG
M A L S V L R L A L L L L A V T F

130 140 150 160 170 180
CAGCCTCTCTGATCCCTCAATTTGGTCTGTTTTCAAAATATAGAACGCCAAACTGCTCTC
A A S L I P Q F G L F S K Y R T P N C S
190 200 210 220 230 240
AGTATAGATTACCAGGATGTCCCAGACACTTTAACCCTGTGTGTGGCAGTGACATGTCCA
Q Y R L P G C P R H F N P V C G S D M S

250 260 270 280 290 300
CTTATGCCAATGAATGTACTCTGTGCATGAAAATCAGGGAAGGTGGTCATAATATTAAAA
T Y A N E C T L C M K I R E G G H N I K

310 320
TCATTCGAAATGGACCCTGC (II)
I I R N G P C

6. DNA nach Anspruch 5 (b'), (c') oder (d'), dadurch gekennzeichnet, daß die Basensequenz (II) am 5'-Terminus um Kodons für ein Leader-Peptid, beispielsweise für eine Signalsequenz oder eine Signalsequenz mit Linkersequenz, verlängert ist.

7. DNA nach Anspruch 5 (c'), dadurch gekennzeichnet, daß bei der Basensequenz (II) das Kodon für die Aminosäure der Position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 und/oder 32 der Sequenz (I) weggelassen ist.

8. DNA nach Anspruch 5 (d'), dadurch gekennzeichnet, daß bei der Basensequenz (II) das Kodon für die Aminosäure der Position 45, 46, 47, 48, 49, 56, 57, 60, 64 und/oder 67 durch ein Kodon für eine andere Aminosäure, vorzugsweise eine andere in der Natur vorkommende Aminosäure, ersetzt ist.

9. Vektor zur Expression eines Peptids gemäß einem der Ansprüche 1 bis 4, dadurch *gekennzeichnet,* daß er eine DNA gemäß einem der Ansprüche 5 bis 8 umfaßt.

10. Vektor nach Anspruch 9, dadurch *gekennzeichnet,* daß es sich um pMAMPF2 oder pMAMPF3 mit einer DNA gemäß einem der Ansprüche 5 bis 8 handelt.

11. Wirtsorganismus, dadurch *gekennzeichnet,* daß er mit einem Vektor gemäß Anspruch 9 oder 10 transformiert ist.

12. Wirtsorganismus nach Anspruch 11, dadurch *gekennzeichnet,* daß es sich um Escherichia coli handelt, insbesondere DSM 5627.

13. Verfahren zur Gewinnung eines Peptids gemäß einem der Ansprüche 1 bis 4, dadurch *gekennzeichnet,* daß man einen Wirtsorganismus gemäß einem der Ansprüche 11 oder 12 verwendet und das Peptid in an sich bekannter Weise gewinnt.

14. Pharmazeutisches Präparat, dadurch gekennzeichnet, daß es aus einem Peptid gemäß einem der Ansprüche 1 bis 4 besteht oder ein derartiges Peptid gegebenenfalls neben einem oder mehreren anderen Wirkstoffen und/oder einem üblichen Träger und/oder Verdünnungsmittel umfaßt.

## Claims

1. (a) Peptide of the following amino acid sequence (I) or (b) peptide comprising the following amino acid sequence (I) or (c) peptide of the following amino acid sequence (I) or comprising the following amino acid sequence (I), in which one or more amino acids have been omitted or
(d) peptide of the following amino acid sequence (I) or comprising the following amino acid sequence (I), in which one or more amino acids have each been replaced by a different amino acid: 
10
Met Ala Leu Ser Val Leu Arg Leu Ala Leu Leu Leu Leu Ala Val
20 30
Thr Phe Ala Ala Ser Leu Ile Pro Gln Phe Gly Leu Phe Ser Lys
40
Tyr Arg Thr Pro Asn Cys Ser Gln Tyr Arg Leu Pro Gly Cys Pro
50 60
Arg His Phe Asn Pro Val Cys Gly Ser Asp Met Ser Thr Tyr Ala
70
Asn Glu Cys Thr Leu Cys Met Lys Ile Arg Glu Gly Gly His Asn
80
Ile Lys Ile Ile Arg Asn Gly Pro Cys (I)
 which peptide blocks leukocyte elastase and cathepsin G.

2. Peptide according to claim 1 (b), (c) or (d), characterised in that the amino acid sequence (I) is preceded by a leader peptide, for example a signal sequence or a signal sequence with a linker sequence.

3. Peptide according to claim 1 (c), characterised in that the amino acid at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 and/or 32 of the given amino acid sequence (I) has been omitted.

4. Peptide according to claim 1 (d), characterised in that the amino acid at position 45, 46, 47, 48, 49, 56, 57, 60, 64 and/or 67 has been replaced by a different amino acid, preferably a different naturally occurring amino acid.

5. DNA, characterised in that it
(a') encodes a peptide according to claim 1 (a) and has the following base sequence (II) or a functionally equivalent base sequence, or
(b') encodes a peptide according to claim 1 (b), (c) or (d) and comprises the following base sequence (II) or a functionally equivalent base sequence, or
(c') encodes a peptide according to claim 1 (c) and has or comprises the following base sequence (II) in which one or more codons have been omitted, or a functionally equivalent base sequence, or
(d') encodes a peptide according to claim 1 (d) and has or comprises the following base sequence (II) in which one or more codons have each been replaced by a different codon, or a functionally equivalent base sequence:

70 80 90 100 110 120
ATGGCGCTGTCGGTGCTGCGCTTGGCGCTGCTGCTCCTGGCAGTCACCTTCG
M A L S V L R L A L L L L A V T F

130 140 150 160 170 180
CAGCCTCTCTGATCCCTCAATTTGGTCTGTTTTCAAAATATAGAACGCCAAACTGCTCTC
A A S L I P Q F G L F S K Y R T P N C S

190 200 210 220 230 240
AGTATAGATTACCAGGATGTCCCAGACACTTTAACCCTGTGTGTGGCAGTGACATGTCCA
Q Y R L P G C P R H F N P V C G S D M S

250 260 270 280 290 300
CTTATGCCAATGAATGTACTCTGTGCATGAAAATCAGGGAAGGTGGTCATAATATTAAAA
T Y A N E C T L C M K I R E G G H N I K

310 320
TCATTCGAAATGGACCCTGC (II)
I I R N G P C

6. DNA according to claim 5 (b'), (c') or (d'), characterised in that the base sequence (II) has been extended at the 5' terminus by codons for a leader peptide, for example for a signal sequence or a signal sequence with a linker sequence.

7. DNA according to claim 5 (c'), characterised in that in the base sequence (II) the codon for the amino acid at position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 and/or 32 of sequence (I) has been omitted.

8. DNA according to claim 5 (d'), characterised in that in the base sequence (II) the codon for the amino acid at position 45, 46, 47, 48, 49, 56, 57, 60, 64 and/or 67 has been replaced by a codon for a different amino acid, preferably a different naturally occurring amino acid.

9. Vector for the expression of a peptide according to any one of claims 1 to 4, characterised in that it comprises a DNA according to any one of claims 5 to 8.

10. Vector according to claim 9, characterised in that it is pMAMPF2 or pMAMPF3 together with a DNA according to any one of claims 5 to 8.

11. Host organism, characterised in that it has been transformed with a vector according to claim 9 or 10.

12. Host organism according to claim 11, characterised in that it is Escherichia coli, especially DSM 5627.

13. Process for obtaining a peptide according to any one of claims 1 to 4, characterised in that a host organism according to either of claims 11 and 12 is used and the peptide is obtained in a manner known per se.

14. Pharmaceutical preparation, characterised in that it consists of a peptide according to any one of claims 1 to 4 or comprises such a peptide, where appropriate together with one or more other active ingredients and/or a customary carrier and/or diluent.

## Revendications

1. (a) Peptide comportant la séquence d'acides aminés (I) suivante, ou
(b) peptide renfermant la séquence d'acides aminés (I) suivante, ou
(c) peptide comportant la séquence d'acides aminés (I) suivante, ou renfermant la séquence d'acides aminés (I) suivante, de laquelle on a éliminé un ou plusieurs acides aminés, ou
(d) peptide comportant la séquence d'acides aminés (I) suivante ou renfermant la séquence d'acides aminés (I) suivante, dans laquelle on a échangé un ou plusieurs acides aminés chacun contre un autre acide aminé 
10
Met Ala Leu Ser Val Leu Arg Leu Ala Leu Leu Leu Leu Ala Val

20 30
Thr Phe Ala Ala Ser Leu Ile Pro Gln Phe Gly Leu Phe Ser Lys

40
Tyr Arg Thr Pro Asn Cys Ser Gln Tyr Arg Leu Pro Gly Cys Pro

50 60
Arg His Phe Asn Pro Val Cys Gly Ser Asp Met Ser Thr Tyr Ala

70
Asn Glu Cys Thr Leu Cys Met Lys Ile Arg Glu Gly Gly His Asn

80
Ile Lys Ile Ile Arg Asn Gly Pro Cys (I)
le peptide bloquant l'élastase leucocytaire et la cathepsine G.

2. Peptide selon la revendication 1 (b), (c) ou (d), caractérisé en ce que la séquence d'acides aminés (I) précède le peptide de tête, par exemple une séquence signal ou une séquence signal avec une séquence de liaison.

3. Peptide selon la revendication 1 (c), caractérisé en ce qu'on supprime les acides aminés des positions 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 et/ou 32 de la séquence d'acides aminés (I) indiquée.

4. Peptide selon la revendication 1 (d), caractérisé en ce qu'on remplace les acides aminés des positions 45, 46, 47, 48, 49, 56, 57, 60, 64 et/ou 67 par un autre acide aminé, de préférence par un autre acide aminé qui se trouve dans la nature.

5. ADN, *caractérisé* en ce qu'il
(a') code pour un peptide selon la revendication 1 (a) et présente la séquence de base (II) suivante ou une séquence de base fonctionnellement équivalente, ou
(b') code pour un peptide conformément à la revendication 1 (b), (c) ou (d), et renferme les séquences de base (II) suivantes ou une séquence de base fonctionnellement équivalente, ou
(c') code pour un peptide selon la revendication 1 (c), et présente ou renferme la séquence de base (II) suivante, de laquelle on a supprimé un ou plusieurs codons, ou une séquence de base fonctionnellement équivalente, ou
(d') code pour un peptide selon la revendication 1 (d), et présente ou renferme la séquence de base (II) suivante, dans laquelle on a échangé un ou plusieurs codons, chacun contre un autre codon, ou présente ou renferme une séquence de base fonctionnellement équivalente

70 80 90 100 110 120
ATGGCGCTGTCGGTGCTGCGCTTGGCGCTGCTGCTCCTGGCAGTCACCTTCG
M A L S V L R L A L L L A V T F

130 140 150 160 170 180
CAGCCTCTCTGATCCCTCAATTTGGTCTGTTTTCAAAATATAGAACGCCAAACTGCTCTC
A A S L I P Q F G L F S K Y R T P N C S
190 200 210 220 230 240
AGTATAGATTACCAGGATGTCCCAGACACTTTAACCCTGTGTGTGGCAGTGACATGTCCA
Q Y R L P G C P R H F N P V C G S D M S

250 260 270 280 290 300
CTTATGCCAATGAATGTACTCTGTGCATGAAAATCAGGGAAGGTGGTCATAATATTAAAA
T Y A N E C T L C M K I R E G G H N I K
310 320
TCATTCGAAATGGACCCTGC (II)
I I R N G P C

6. ADN selon la revendication 5 (b'), (c') ou (d'), caractérisé en ce que la séquence de base (II) est prolongée à l'extrémité 5' par des codons pour une séquence peptidique de tête, par exemple pour une séquence signal ou par une séquence signal avec une séquence de liaison.

7. ADN selon la revendication 5 (c'), caractérisé en ce qu'on supprime dans la séquence de base (II) le codon pour les acides aminés dans les positions 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 et/ou 32 de la séquence (I).

8. ADN selon la revendication 5 (d'), caractérisé en ce qu'on remplace dans la séquence de base (II) le codon pour les acides aminés des positions 45, 46, 47, 48, 49, 56, 57, 60, 64 et/ou 67 par un codon pour un autre acide aminé, de préférence un autre acide aminé qui se trouve dans la nature.

9. Vecteur d'expression d'un peptide selon l'une des revendications 1 à 4, *caractérisé* en ce qu'il comporte un ADN selon l'une des revendications 5 à 8.

10. Vecteur selon la revendication 9, *caractérisé* en ce qu'il s'agit de pMAMPF2 ou bien pMAMPF3 avec un ADN selon l'une des revendications 5 à 8.

11. Organisme hôte, *caractérisé* en ce qu'il est transformé par un vecteur selon la revendication 9 ou 10.

12. Organisme hôte, selon la revendication 11, *caractérisé* en ce qu'il s'agit d'Escherichia coli, plus particulièrement de DSM 5627.

13. Procédé pour l'obtention d'un peptide selon l'une des revendications 1 à 4, caractérisé en ce qu'on utilise un micro-organisme hôte selon l'une des revendications 11 ou 12, et on obtient le peptide de façon connue en soi.

14. Préparation pharmaceutique, caractérisée en ce qu'elle se compose d'un peptide selon l'une des revendications 1 à 4, ou renferme un tel peptide, éventuellement aux côtés d'un ou plusieurs principes actifs et/ou un support et/ou un diluant usuels.
